# EUROPEAN PATENT SPECIFICATION

(11) **EP 4 327 765 B1**
(45) Date of publication and mention of the grant of the patent: **19.03.2025**
(21) Application number: 23193016.5
(22) Date of filing: 23.08.2023
(51) Int. Cl.: A61B 17/34

(54) **EXPANDABLE CANNULA ASSEMBLY**
EXPANDIERBARE KANÜLENANORDNUNG
ENSEMBLE CANULE EXTENSIBLE

(30) Priority: 23.08.2022 US 202263400111 P; 22.08.2023 US 202318236787
(43) Date of publication of application: 28.02.2024
(73) Proprietor: LSI Solutions, Inc., Victor, NY 14564 (US)
(72) Inventor: SAUER, Jude S., Pittsford, 14534 (US)
(74) Representative: Flügel Preissner Schober Seidel

(56) References cited:
- US-A1- 2005 075 644
- US-A1- 2005 149 106
- US-A1- 2006 229 636
- US-A1- 2012 029 296
- US-A1- 2012 130 161

## Description

### FIELD OF THE INVENTION

The claimed invention relates to surgical devices, and more specifically to a cannula assembly.

### BACKGROUND OF THE INVENTION

Cannulas are typically used during minimally-invasive surgical procedures to insert one or more instruments through an incision through a patient to a treatment area of the patient. The cannula typically guides the one or more instruments to the treatment area and allows the one or more instrument to pass through the incision without additional trauma to the incision area. Because the instruments used in the procedure may sometimes have large distal ends, the diameter or cross-sectional shape of the cannula must be large enough to accommodate the largest of such instruments. However, such a relatively large cross-sectional shape of the cannula requires a large incision in the patient, and large incision is maintained as long as the cannula passes through the incision of the patient. The resulting relatively large incision size needed to accommodate the cannula results in increased recovery time for a patient, but can also lead to pain, an increased risk of infection, and other disadvantageous outcomes for the patient during recovery. Accordingly, there is a need for a cannula that can accommodate large instrument during a procedure but minimizes the trauma to the incision area.

US 2006/229636 A1 discloses a device tor providing access to a surgical site in a body. The device comprises and expandable portion including first and second members. Each member includes a receiving portion and a flap.

US 2005/149106 A1 discloses a cannula for receiving surgical instruments for performing a surgical procedure including a second tubular portion. The second tubular portion comprises one arcuate slot in each segment having one guide member of another segment slidably arranged within the slots. The second tubular portion of the tubular structure is expandable from a contracted condition to an expanded condition.

US 2005/075644 A1 discloses an access device. The access device comprises two skirt portions. The skirt portion comprises two slots which are connected to each other by use of a rivet. The rivet is permitted to move freely within the slots to expand and contract the access device.

US 2012/029296 A1 discloses an expandable cannula assembly for use in percutaneous surgical procedures.

US 2012/130161 A1 discloses a cannula having a dynamically expandable working channel.

### SUMMARY OF THE INVENTION

The invention is set out in the appended claims.

### BRIEF DESCRIPTION OF THE DRAWINGS

Figures 1A to 2D are various views of an embodiment of an expandable cannula assembly in a first position;
Figure 3A is a cross-sectional view of the embodiment of the expandable cannula assembly of Figure 1B taken along section line 3A-3A;
Figure 3B is a perspective view of the cross-sectional view of Figure 3A;
Figure 3C is a cross-sectional view of the embodiment of the expandable cannula assembly of Figure 1B taken along section line 3C-3C;
Figure 3D is a perspective view of the cross-sectional view of Figure 3C;
Figures 4A to 4C are various views of an embodiment of a first cannula subassembly of the expandable cannula assembly;

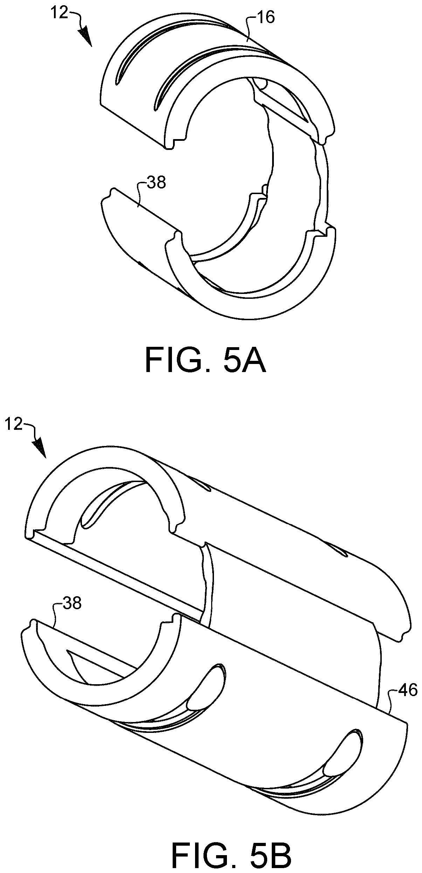

Figures 5A and 5B are various views of the embodiment of the first cannula subassembly of the expandable cannula assembly coupled with the lower body member of the second cannula subassembly, with the lower lateral panel of the second cannula subassembly omitted for clarity;
Figures 6A to 6C are various views of an embodiment of a second cannula subassembly of the expandable cannula assembly;
Figure 6D is a side view of a second protrusion of a lower lateral panel of the second cannula subassembly;
Figure 7 is a perspective view of the embodiment of the second cannula subassembly of the expandable cannula assembly coupled with the upper body member of the first cannula subassembly, with the upper lateral panel of the first cannula subassembly omitted for clarity;
Figure 8A is a front view of the embodiment of the first cannula subassembly and the second cannula subassembly of the expandable cannula assembly in a second position;
Figure 8B is a front view of the embodiment of the first cannula subassembly and the second cannula subassembly of the expandable cannula assembly in a third position between the second position and the first position;
Figure 8C is a perspective view of the embodiment of the first cannula subassembly and the second cannula subassembly of the expandable cannula assembly in the first position;
Figures 9A to 9B are top and side views, respectively, of an embodiment of an upper body member of the first cannula subassembly;
Figure 9C is a cross-sectional view of the embodiment of the upper body member of Figure 9B taken along section line 9C-9C;
Figure 9D to 9F are perspective views of the embodiment of the upper body member of Figure 9B;
Figures 10A to 10B are top and side views, respectively, of an embodiment of a lower body member of the second cannula subassembly;
Figure 10C is a cross-sectional view of the embodiment of the lower body member of Figure 10A taken along section line 10C-10C;
Figure 10D is a perspective view of the cross-section of the lower body member of Figure 10A taken along section line 10D-10D; and
Figure 11 is a side view of a distal end of a medical instrument that is to be inserted into an end of an embodiment of the expandable cannula assembly in the first position, with the expandable cannula assembly maintained in the first position prior to the insertion of the distal end of the instrument for illustrative reasons.

### DETAILED DESCRIPTION OF THE INVENTION

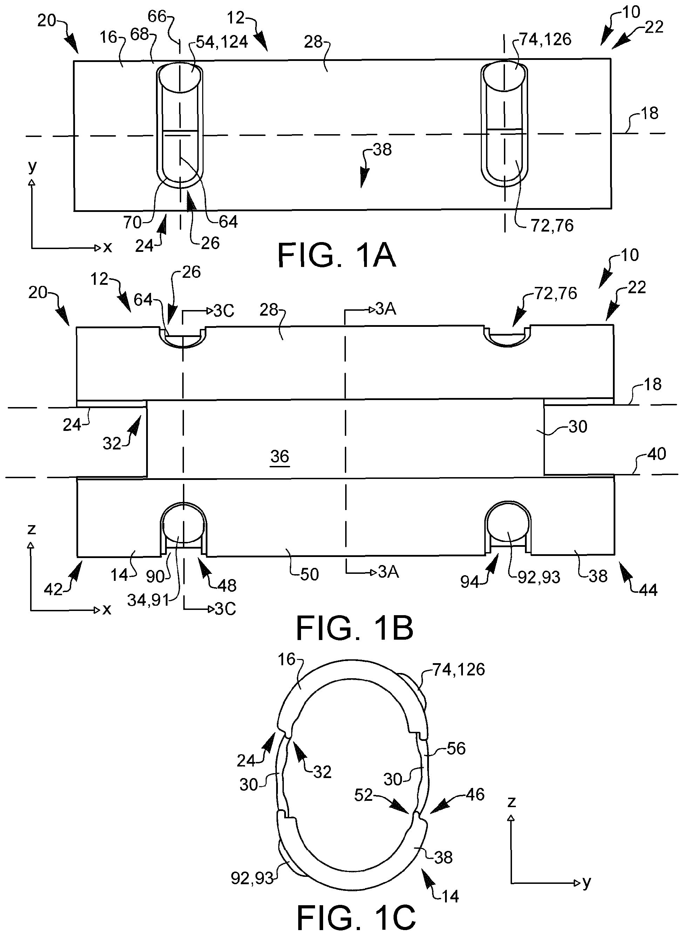

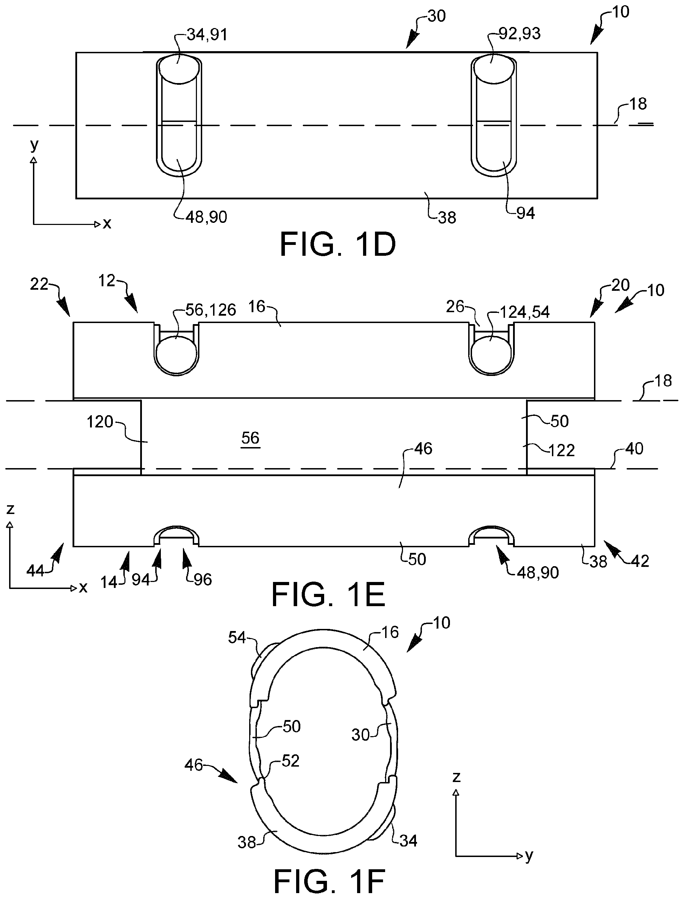

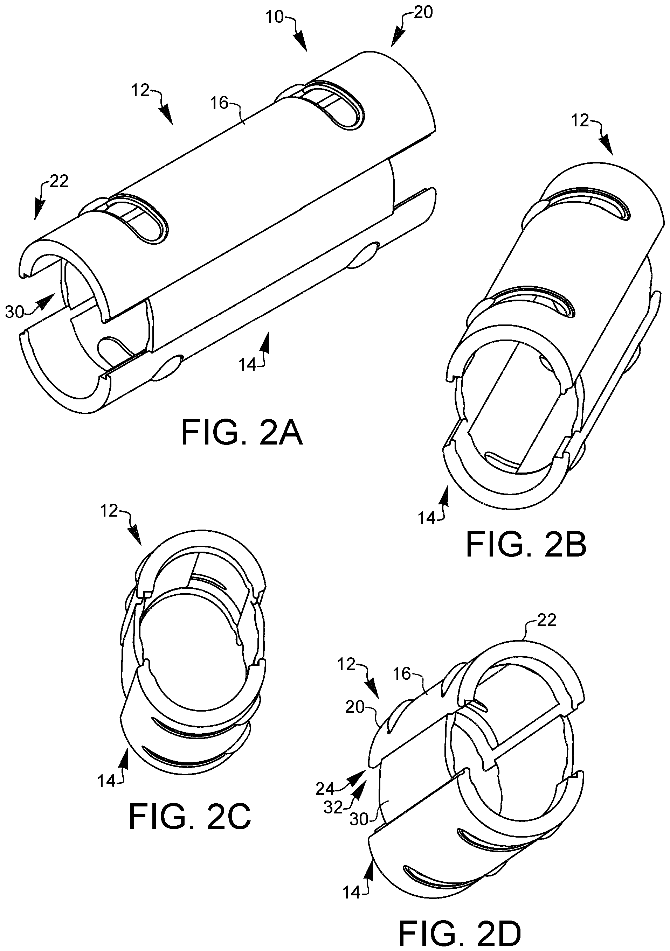

As illustrated in Figures 1A to 2D, an embodiment of an expandable cannula assembly 10 includes a first cannula subassembly 12 and a second cannula subassembly 14 that is movably coupled to the first cannula subassembly 12. With reference to Figure 1B, the first cannula subassembly 12 includes an upper body member 16 that extends along an upper body axis 18 from a first end 20 to a second end 22, the upper body member 16 having a first body edge portion 24 that extends parallel to and offset from the upper body axis 18, and a first upper body mating feature 26 is disposed along a first portion of a surface 28 of the upper body member 16. The first cannula subassembly 12 also includes an upper lateral panel 30 having a first panel edge 32 (see Fig. 1C) that is coupled to the upper body member 16 at or adjacent to the first body edge portion 24 of the upper body member 16, and a first upper lateral panel mating feature 34 is disposed on a first portion of a surface 36 of the upper lateral panel 30.

Still referring to Figure 1B, the second cannula subassembly 14 includes a lower body member 38 that extends along a lower body axis 40 from a first end 42 to a second end 44. With reference to Fig. 1E, the lower body member 38 having a first body edge portion 46 that extends parallel to and offset from the lower body axis 40, wherein a first lower body mating feature 48 is disposed along a first portion of a surface 50 of the lower body member 38. The second cannula subassembly 14 also includes a lower lateral panel 50 having a first panel edge 52 that is coupled to the lower body member 38 at or adjacent to the first body edge portion 46 of the lower body member 38 (see Fig. 1F), and a first lower lateral panel mating feature 54 is disposed on a first portion of a surface 56 of the lower lateral panel 50.

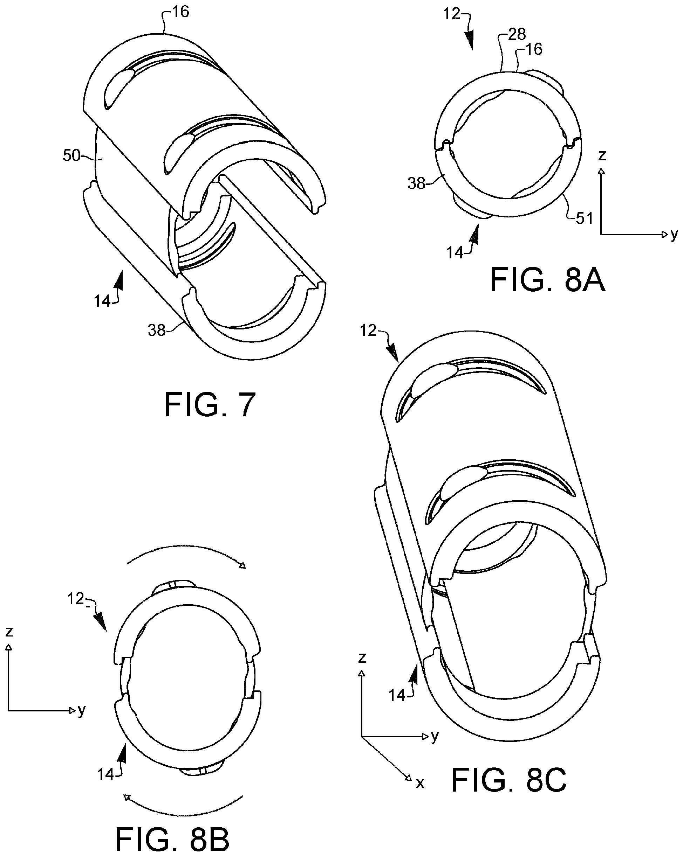

As illustrated in Fig. 1E, the first lower lateral panel mating feature 54 operatively engages the first upper body mating feature 26 such that the first lower lateral panel mating feature 54 is displaceable relative to the first upper body mating feature 26. With reference to Fig. 1B, the first upper lateral panel mating feature 34 operatively engages the first lower body mating feature 48 such that the first upper lateral panel mating feature 34 is displaceable relative to the first lower body mating feature 48. So configured, the first cannula subassembly 12 and the second cannula assembly 14 are displaceable between a first position (or a deployed position, illustrated in Figs. 1A to 2D) and a second position (or an undeployed position, illustrated in Fig. 8A). In the first position, the first cannula subassembly 12 and the second cannula assembly 14 have a first cross-sectional assembly shape and in the second position, the first cannula subassembly 12 and the second cannula assembly 14 have a second cross-sectional assembly shape that is different than the first cross-sectional shape.

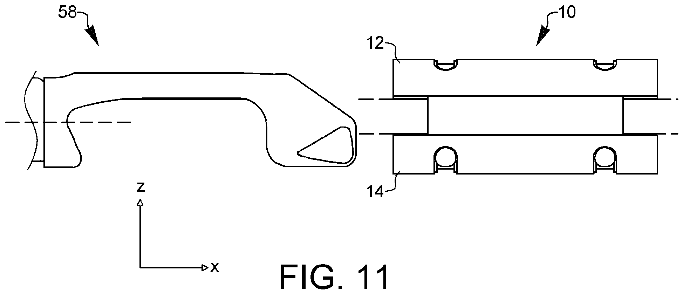

In use during a procedure, the expandable cannula assembly 10 may be inserted into an incision of a patient in the second position to minimize trauma to the incision at the area of insertion. However, if a relatively large instrument (such as the instrument 58 illustrated in Fig. 11) must be passed through the expandable cannula assembly 10, the first cannula subassembly 12 and the second cannula assembly 14 may transition from the second position into the "deployed position," or the first position, to accommodate the large instrument 58, and the transition from the second position to the first position will be discussed in more detail in a following section. When the instrument 58 is removed from the expandable cannula assembly 10, the first cannula subassembly 12 and the second cannula assembly 14 may quickly transition back to the second position, thereby minimizing the time in which the expandable cannula assembly 10 is in the more traumatic first position.

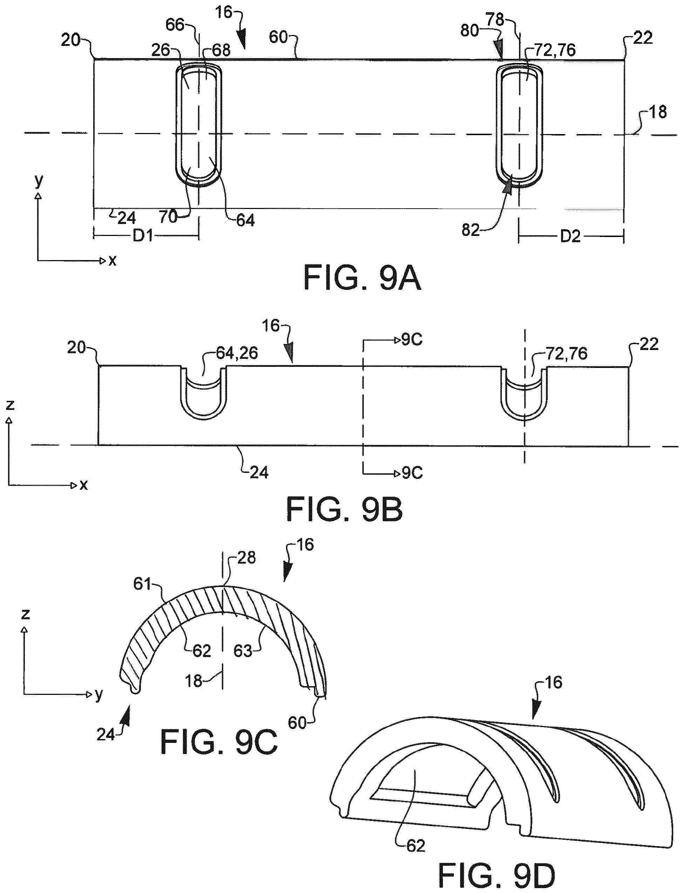

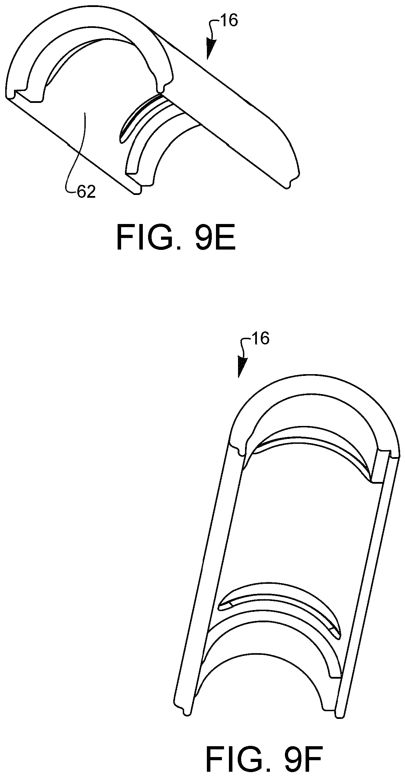

Turning to the first cannula subassembly 12 of the expandable cannula assembly 10 in more detail, Fig. 9A illustrates the upper body member 16 that extends along the upper body axis 18 from the first end 20 to the second end 22. The upper body axis 18 extends parallel to or along the X-axis of the reference coordinate system of Figs. 9A to 9C. The upper body member 16 may have any suitable cross-sectional shape or combination of shapes. In particular, when viewed in a cross-section that is normal to the X-axis, such as a cross-sectional plane that is parallel to the Y-Z plane of the reference coordinate system of Figs. 9A to 9C, the upper body member 16 may have an arcuate, semi-circular, or partially-circular cross-sectional shape. That is, in cross section, the upper body member 16 may extend from the first body edge portion 24 to a second body edge portion 60 along a path or axis that may be a segment of a circle, such as one half of a circle, as illustrated in Fig. 9C. The cross-sectional shape may be further defined by a first edge 61 defined by the surface 28 (for example, the exterior surface) and second edge 63 defined by an interior surface 62. The cross-sectional shape may be bisected by a plane that contains the upper body axis 18 and that is parallel to the X-Z plane of the reference coordinate system of Figs. 9A to 9C. The cross-sectional shape may be uniform or substantially uniform along the upper body axis 18 from the first end 20 of the upper body member 16 to the second end 22 of the upper body member 16.

As illustrated in Fig. 9A, the upper body member 16 includes the first upper body mating feature 26 that is disposed along the first portion of the surface 28 of the upper body member 16. The first upper body mating feature 26 engages the first lower lateral panel mating feature 54 such that the first lower lateral panel mating feature 54 is displaceable relative to the first upper body mating feature 26. The first upper body mating feature 26 is a first slot 64 disposed along the first portion of the surface 28 of the upper body member 16, and the first slot 64 of the upper body member 16 extends along a first slot axis 66 from a first end 68 to a second end 70. The first slot axis 66 may extend parallel to the Y-axis of the reference coordinate system of Figs. 9A to 9C when viewed along the Z-axis. However, the first slot axis 66 may conform in shape to the cross-sectional shape of the upper body member 16 when viewed in a cross-section that is normal to the X-axis, such as a cross-sectional plane that is parallel to the Y-Z plane of the reference coordinate system of Figs. 9A to 9C. The first slot 64 may be defined by first and second lateral edges that are parallel, and the first and second ends 68, 70 may be defined by a first and second semi-circular edge, respectively. The first slot 64 may be disposed any distance from the first end 20 of the upper body member 16. In particular, the first slot axis 66 may be disposed a first distance D1 from the first end 20 of the upper body member 16.

Still referring to Fig. 9A, the upper body member 16 includes a second upper body mating feature 72 that is disposed along a second portion of the surface 28 of the upper body member 16. The second upper body mating feature 72 engages a second lower lateral panel mating feature 74 such that the second lower lateral panel mating feature 74 is displaceable relative to the second upper body mating feature 72. In some embodiments, the second upper body mating feature 72 may be identical to the first upper body mating feature 26. Specifically, the second upper body mating feature 72 is a second slot 76 disposed along the second portion of the surface 28 of the upper body member 16, and the second slot 76 of the upper body member 16 extends along a first slot axis 78 from a first end 80 to a second end 82. The second slot axis 78 may extend parallel to the Y-axis of the reference coordinate system of Figs. 9A to 9C (and to the first slot axis 66) when viewed along the Z-axis. However, identical to the first slot axis 66, the second slot axis 78 may conform in shape to the cross-sectional shape of the upper body member 16 when viewed in a cross-section that is normal to the X-axis, such as a cross-sectional plane that is parallel to the Y-Z plane of the reference coordinate system of Figs. 9A to 9C. The second slot axis 78 may be defined by first and second lateral edges that are parallel, and the first and second ends 80, 82 may be defined by a first and second semi-circular edge, respectively. The second slot axis 78 may be disposed any distance from the second end 22 of the upper body member 16. In particular, the second slot axis 78 may be disposed a second distance D2 from the second end 22 of the upper body member 16, and the second distance D2 may be equal to the first distance D1 between the first slot axis 66 and the first end 20 of the upper body member 16.

As illustrated in Fig. 1B, the first cannula subassembly 12 also includes the upper lateral panel 30 having a first panel edge 32 (see Fig. 1C) disposed at a first end of the upper lateral panel 30, and the first panel edge 32 (or any portion of the first end of the upper lateral panel 30) may be coupled to the upper body member 16 at or adjacent to the first body edge portion 24 of the upper body member 16. The first panel edge 32 of the upper lateral panel 30 may be coupled to the upper body member 16 in any suitable manner. For example, the first panel edge 32 of the upper lateral panel 30 may be fixedly coupled to the upper body member 16 by an adhesive, ultrasonic welding, or by mechanical coupling, such as snap-fit or friction fit coupling. In other embodiments, the first panel edge 32 of the upper lateral panel 30 may be coupled to the upper body member 16 by a hinge or flexible coupling. In still further embodiments, the first panel edge 32 of the upper lateral panel 30 may be integrally formed with the upper body member 16 at or adjacent to the first body edge portion 24 of the upper body member 16.

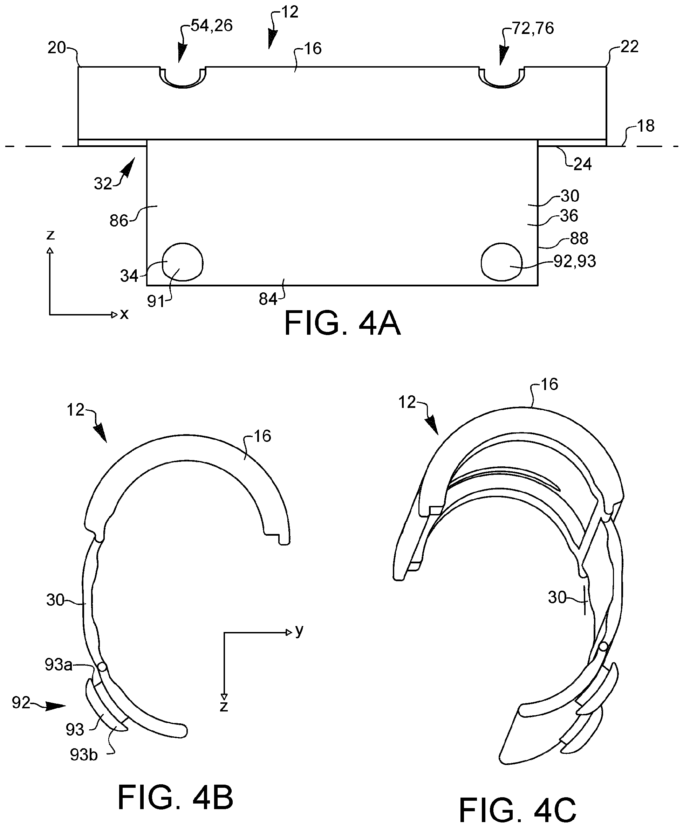

The upper lateral panel 30 may have any suitable shape or combination of shapes. In the embodiment of Fig. 4A, the upper lateral panel 30 may be defined by the first panel edge 32 and a second panel edge 84 that may be parallel (or substantially parallel) to the first panel edge 32 and/or the X-axis of the reference coordinate system of Figs. 1B and 4A. The second panel edge 84 may define a second end of the upper lateral panel 30. The upper lateral panel 30 may be further defined by a first lateral edge 86 and a second lateral edge 88, where the first lateral edge 86 extends from a first end of the first panel edge 32 to a first end of the second panel edge 84 and the second lateral edge 88 extends from a second end of the first panel edge 32 to a second end of the second panel edge 84. Each of the first lateral edge 86 and the second lateral edge 88 may extend normal to the X-axis of the reference coordinate system of Figs. 1B and 4A *(i.e.,* along or parallel to the Z-axis). Accordingly, the upper lateral panel 30 may have a square or rectangular shape (or a substantially square or rectangular shape).

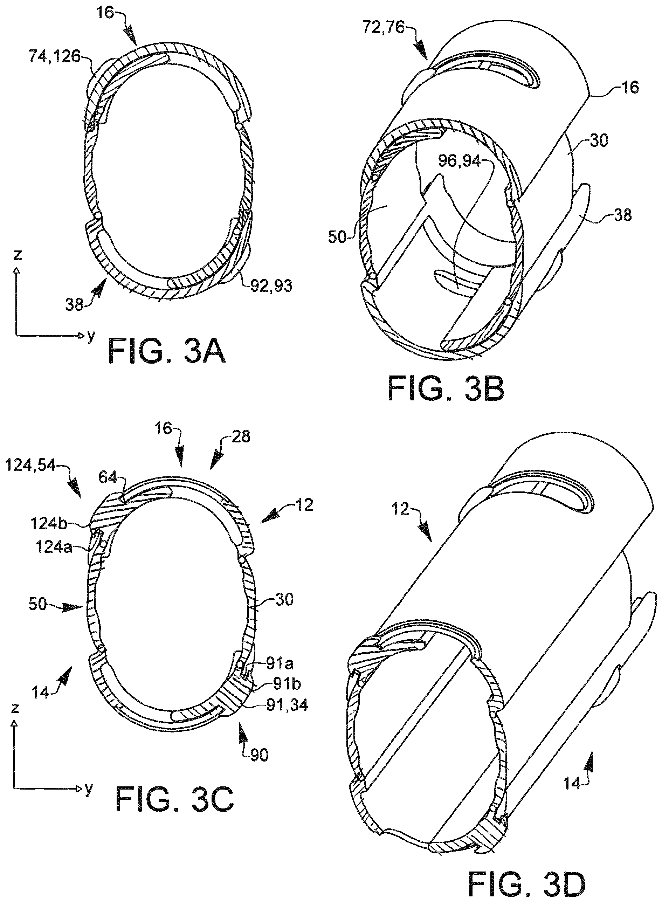

The upper lateral panel 30 has the first upper lateral panel mating feature 34 that is disposed on the first portion of the surface 36 *(i.e.,* the outside surface) of the upper lateral panel 30. The first upper lateral panel mating feature 34 engages the first lower body mating feature 48 of the lower body member 38 (illustrated in Fig. 1B) such that the first upper lateral panel mating feature 34 is displaceable relative to the first lower body mating feature 48. The first upper lateral panel mating feature 34 is a first protrusion 91 that is adapted to be received in a first slot 90 disposed along the surface 50 of the lower body member 38, and the first slot 90 will be described in more detail in a following section. As illustrated in the cross-sectional view of Fig. 3C, the first protrusion 91 may have a base portion 91a that may be cylindrical and may have a diameter that is slightly smaller than the distance between the lateral edges of the first slot 90 such that the first protrusion 91 may translate longitudinally within the first slot 90. The first protrusion 91 may also have a top portion 91b coupled to the base portion 91a, and the top portion 91b may have the shape of a disk and may have a diameter that is larger than the distance between the lateral edges of the first slot 90 such that the first protrusion 91 is retained in the first slot 90 as the first protrusion 91 translates longitudinally within the first slot 90. The first protrusion 91 may be disposed adjacent to the second panel edge 84 (or the second end) of the upper lateral panel 30 and may be positioned to align with the first lower body mating feature 48 of the lower body member 38.

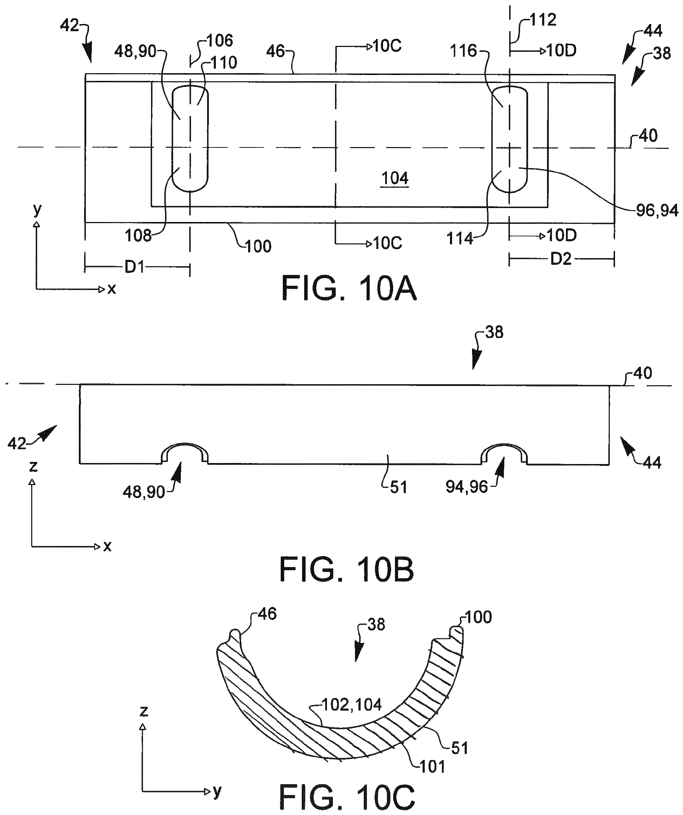

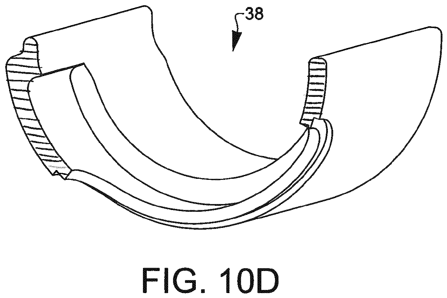

The upper lateral panel 30 may also have a second upper lateral panel mating feature 92 that is disposed on a second portion of the surface 36 (i.e., the outside surface) of the upper lateral panel 30. The second upper lateral panel mating feature 92 may be any feature that may engage a second lower body mating feature 96 of the lower body member 38 (illustrated in Fig. 1B) such that the second upper lateral panel mating feature 92 is displaceable relative to the second lower body mating feature 96. In some embodiments, the second upper lateral panel mating feature 92 may be identical to the first upper lateral panel mating feature 34. For example, the second upper lateral panel mating feature 92 may be a second protrusion 93 that that may be adapted to be received in a second slot 94 (see Fig. 1B) disposed along a second portion of the surface 50 of the lower body member 38, and the second slot 94 may be identical to the first slot 90 and will be described in more detail in a following section. As illustrated in Fig. 4B, the second protrusion 93 may be identical to the first protrusion 91 and may have a base portion 93a that may be cylindrical and may have a diameter that is slightly smaller than the distance between the lateral edges of the second slot 94 (see Fig. 10A) such that the second protrusion 93 may translate longitudinally within the second slot 94. The second protrusion 93 may also have a top portion 93b coupled to the base portion 93a, and the top portion 93b may have the shape of a disk and may have a diameter that is larger than the distance between the lateral edges of the second slot 94 (see Fig. 10A) such that the second protrusion 93 is retained in the second slot 94 as the second protrusion 93 translates longitudinally within the second slot 94. As illustrated in Fig. 4A, the second protrusion 93 may be disposed adjacent to the second panel edge 84 (or the second end) of the upper lateral panel 30 and may be positioned to align with the second lower body mating feature 96 of the lower body member 38.

The upper lateral panel 30 may be made from any suitable material, such as a flexible material. In some embodiments, the upper lateral panel 30 is configured to deform along an axis that is parallel to the upper body axis 18. In some embodiments, the upper lateral panel 30 may be made from a resilient material that may deform in a direction normal to the parallel to the upper body axis 18.

Turning to the second cannula subassembly 14 of the expandable cannula assembly 10 in more detail, Fig. 10A illustrates the lower body member 38 that may be identical (or similar) to the upper body member 16. In particular, the lower body member 38 may extend along the lower body axis 40 from the first end 42 to the second end 44. The lower body axis 40 extends parallel to or along the X-axis of the reference coordinate system of Figs. 10A to 10C. The lower body member 38 may have any suitable cross-sectional shape or combination of shapes. In particular, when viewed in a cross-section that is normal to the X-axis, such as a cross-sectional plane that is parallel to the Y-Z plane of the reference coordinate system of Figs. 10A to 10C, the lower body member 38 may have an arcuate, semi-circular, or partially-circular cross-sectional shape. That is, in cross section, the lower body member 38 may extend from the first body edge portion 46 to a second body edge portion 100 along a path or axis that may be a segment of a circle, such as one half of a circle, as illustrated in Fig. 10C. The cross-sectional shape may be further defined by a first edge 101 defined by the surface 51 (for example, the exterior surface) and second edge 102 defined by an interior surface 104. The cross-sectional shape may be bisected by a plane that contains the lower body axis 40 and that is parallel to the X-Z plane of the reference coordinate system of Figs. 10A to 10C. The cross-sectional shape may be uniform or substantially uniform along the lower body axis 40 from the first end 42 of the lower body member 38 to the second end 44 of the lower body member 38.

As illustrated in Fig. 10A, the lower body member 38 may include the first lower body mating feature 48 that may be disposed along the first portion of the surface 51 of the lower body member 38. The first lower body mating feature 48 may be any feature that may engage the first upper lateral panel mating feature 34 (see Fig. 4A) such that the first upper lateral panel mating feature 34 is displaceable relative to the first lower body mating feature 48. The first lower body mating feature 48 is a first slot 90 disposed along the first portion of the surface 51 of the lower body member 38, and the first slot 90 of the lower body member 38 extends along a first slot axis 106 from a first end 108 to a second end 110. The first slot axis 106 may extend parallel to the Y-axis of the reference coordinate system of Figs. 10A to 10C when viewed along the Z-axis. However, the first slot axis 106 may conform in shape to the cross-sectional shape of the lower body member 38 when viewed in a cross-section that is normal to the X-axis, such as a cross-sectional plane that is parallel to the Y-Z plane of the reference coordinate system of Figs. 10A to 10C. The first slot 90 may be defined by first and second lateral edges that are parallel, and the first and second ends 108, 110 may be defined by a first and second semi-circular edge, respectively. The first slot 90 may be disposed any distance from the first end 42 of the lower body member 38. In particular, the first slot axis 106 may be disposed a first distance D1 from the first end 42 of the lower body member 38.

Still referring to Fig. 10A, the lower body member 38 may include a second lower body mating feature 96 that may be disposed along a second portion of the surface 51 of the lower body member 38. The second lower body mating feature 96 may be any feature that may engage a second upper lateral panel mating feature 92 (see Fig. 4A) such that the second upper lateral panel mating feature 92 is displaceable relative to the second lower body mating feature 96. In some embodiments, the second lower body mating feature 96 may be identical to the first lower body mating feature 48. Specifically, the second lower body mating feature 96 may be the second slot 94 disposed along the second portion of the surface 51 of the lower body member 38, and the second slot 94 of the lower body member 38 may extend along a second slot axis 112 from a first end 114 to a second end 116. The second slot axis 112 may extend parallel to the Y-axis of the reference coordinate system of Figs. 10A to 10C (and to the first slot axis 106) when viewed along the Z-axis. However, identical to the first slot axis 106, the second slot axis 112 may conform in shape to the cross-sectional shape of the lower body member 38 when viewed in a cross-section that is normal to the X-axis, such as a cross-sectional plane that is parallel to the Y-Z plane of the reference coordinate system of Figs. 10A to 10C. The second slot axis 112 may be defined by first and second lateral edges that are parallel, and the first and second ends 114, 116 may be defined by a first and second semi-circular edge, respectively. The second slot axis 112 may be disposed any distance from the second end 44 of the lower body member 38. In particular, the second slot axis 112 may be disposed a second distance D2 from the second end 44 of the lower body member 38, and the second distance D2 may be equal to the first distance D1 between the first slot axis 106 and the first end 42 of the lower body member 38.

As illustrated in Fig. 1B, the first cannula subassembly 12 also includes the lower lateral panel 50 having a first panel edge 52 (see Fig. 1C) disposed at a first end of the lower lateral panel 50, and the first panel edge 52 (or any portion of the first end of the lower lateral panel 50) may be coupled to the lower body member 38 at or adjacent to the first body edge portion 46 of the lower body member 38. The first panel edge 52 of the lower lateral panel 50 may be coupled to the lower body member 38 in any suitable manner. For example, the first panel edge 52 of the lower lateral panel 50 may be fixedly coupled to the lower body member 38 by an adhesive, ultrasonic welding, or by mechanical coupling, such as snap-fit or friction fit coupling. In other embodiments, the first panel edge 52 of the lower lateral panel 50 may be coupled to the lower body member 38 by a hinge or flexible coupling. In still further embodiments, the first panel edge 52 of the lower lateral panel 50 may be integrally formed with the lower body member 38 at or adjacent to the first body edge portion 46 of the lower body member 38.

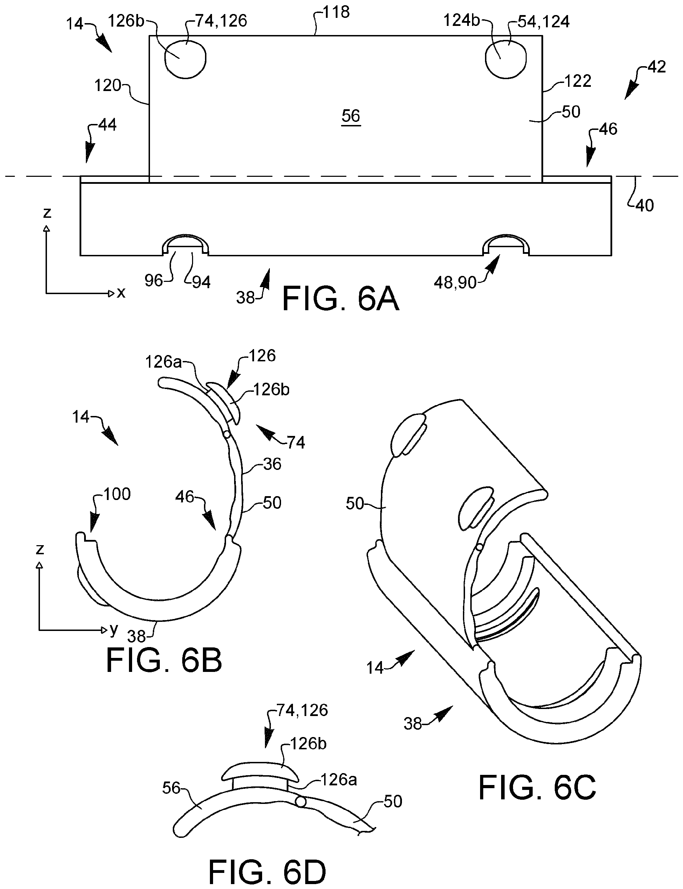

The lower lateral panel 50 may have any suitable shape or combination of shapes. In the embodiment of Fig. 6A, the lower lateral panel 50 may be defined by the first panel edge 52 and a second panel edge 118 that may be parallel (or substantially parallel) to the first panel edge 52 and/or the X-axis of the reference coordinate system of Figs. 1E and 6A. The second panel edge 118 may define a second end of the lower lateral panel 50. The lower lateral panel 50 may be further defined by a first lateral edge 120 and a second lateral edge 122, where the first lateral edge 120 extends from a first end of the first panel edge 52 to a first end of the second panel edge 118 and the second lateral edge 122 extends from a second end of the first panel edge 52 to a second end of the second panel edge 118. Each of the first lateral edge 120 and the second lateral edge 122 may extend normal to the X-axis of the reference coordinate system of Figs. 1E and 6A (i.e., along or parallel to the Z-axis). Accordingly, the lower lateral panel 50 may have a square or rectangular shape (or a substantially square or rectangular shape).

The lower lateral panel 50 may also have the first lower lateral panel mating feature 54 that is disposed on the first portion of the surface 56 (i.e., the outside surface) of the lower lateral panel 50. The first lower lateral panel mating feature 54 may be any feature that may engage the first upper body mating feature 26 of the upper body member 16 (illustrated in Figs. 9A and 9B) such that the first lower lateral panel mating feature 54 is displaceable relative to the first upper body mating feature 26. In some embodiments, the first lower lateral panel mating feature 54 may be identical (or similar) to the first upper lateral panel mating feature 34 (illustrated in Fig. 4A). The first lower lateral panel mating feature 54 is a first protrusion 124 that is adapted to be received in the first slot 64 disposed along the surface 28 of the upper body member 16 (illustrated in Fig. 1A). As illustrated in the cross-sectional view of Fig. 3C, the first protrusion 124 may have a base portion 124a that may be cylindrical and may have a diameter that is slightly smaller than the distance between the lateral edges of the first slot 64 such that the first protrusion 124 may translate longitudinally within the first slot 64. The first protrusion 124 may also have a top portion 124b coupled to the base portion 124a, and the top portion 124b may have the shape of a disk and may have a diameter that is larger than the distance between the lateral edges of the first slot 64 such that the first protrusion 124 is retained in the first slot 64 as the first protrusion 124 translates longitudinally within the first slot 64. The first protrusion 124 may be disposed adjacent to the second panel edge 118 (or the second end) of the lower lateral panel 50 and may be positioned to align with the first upper body mating feature 26 of the upper body member 16.

The lower lateral panel 50 may also have a second lower lateral panel mating feature 74 that is disposed on a second portion of the surface 56 (i.e., the outside surface) of the lower lateral panel 50. The second lower lateral panel mating feature 74 may be any feature that may engage the second upper body mating feature 72 of the upper body member 16 (illustrated in Fig. 1A) such that the second lower lateral panel mating feature 74 is displaceable relative to the second upper body mating feature 72. In some embodiments, the second lower lateral panel mating feature 74 may be identical to the first lower lateral panel mating feature 54. For example, the second lower lateral panel mating feature 74 may be a second protrusion 126 that may be adapted to be received in the second slot 76 disposed along the second portion of the surface 28 of the of the upper body member 16. As illustrated in Fig. 6D, the second protrusion 126 may be identical to the first protrusion 124 and may have a base portion 126a that may be cylindrical and may have a diameter that is slightly smaller than the distance between the lateral edges of the second slot 76 (illustrated in Fig. 9A) such that the second protrusion 126 may translate longitudinally within the second slot 76. The second protrusion 126 may also have a top portion 126b coupled to the base portion 126a, and the top portion 126b may have the shape of a disk and may have a diameter that is larger than the distance between the lateral edges of the second slot 76 (illustrated in Fig. 9A) such that the second protrusion 126 is retained in the second slot 76 as the second protrusion 126 translates longitudinally within the second slot 76. The second protrusion 126 may be disposed adjacent to the second panel edge 118 (or the second end) of the lower lateral panel 50 and may be positioned to align with the second upper body mating feature 72 of the upper body member 16.

The lower lateral panel 50 may be made from any suitable material, such as a flexible material. In some embodiments, the lower lateral panel 50 is configured to deform along an axis that is parallel to the lower body axis 40. In some embodiments, the lower lateral panel 50 may be made from a resilient material that may deform in a direction normal to the parallel to the lower body axis 40.

As previously explained, the first cannula subassembly 12 and the second cannula assembly 14 of the expandable cannula assembly 10 are displaceable between the first position (illustrated in Figs. 1A to 2D) and the second position (illustrated in Fig. 8A). In the first position, the first protrusion 124 of the lower lateral panel 50 is disposed at or adjacent to the first end 68 of the first slot 64 of the upper body member 16, and the second protrusion 126 of the lower lateral panel 50 is disposed at or adjacent to the first end 80 of the second slot 76 of the upper body member 16. Also in the first position, the first protrusion 91 of the upper lateral panel 30 is disposed at or adjacent to the first end 108 of the first slot 90 of the lower body member 38, and the second protrusion 93 of the upper lateral panel 30 is disposed at or adjacent to the first end 114 of the second slot 94 of the lower body member 38. So configured, the first body edge portion 24 of the upper body member 16 is disposed offset *(i.e.,* offset along the Z-axis of the reference coordinate system) from the second body edge portion 100 of the lower body member 38 and the second body edge portion 60 of the upper body member 16 is disposed offset (*i.e.,* offset along the Z-axis of the reference coordinate system) from the first body edge portion 46 of the lower body member 38. In this first position (illustrated in Figure 3A, or when viewed along the upper body axis 18, the lower body axis 40, or parallel to the X-axis of the reference coordinate system), the cross-sectional shape of the upper body member 16 and the upper lateral panel 30 (*i.e.,* the first cross-sectional assembly shape of the first cannula subassembly 12) and the cross-sectional shape of the lower body member 38 and the lower lateral panel 50 (*i.e.,* the first cross-sectional assembly shape of the second cannula subassembly 14) cooperate to form a first cross-sectional assembly shape that is an ovular cross-sectional shape or an oblong cross-sectional shape. The first cannula subassembly 12 and the second cannula subassembly 14 may also cooperate to have the shape of an oval or an oblong shape when viewed along the upper body axis 18, the lower body axis 40, or parallel to the X-axis of the reference coordinate system (such as the view of Figures 1C and 1F).

In the second position, the first protrusion 124 of the lower lateral panel 50 is disposed at or adjacent to the second end 70 of the first slot 64 of the upper body member 16, and the second protrusion 126 of the lower lateral panel 50 is disposed at or adjacent to the second end 82 of the second slot 76 of the upper body member 16. Also in the second position, the first protrusion 91 of the upper lateral panel 30 is disposed at or adjacent to the second end 110 of the first slot 90 of the lower body member 38, and the second protrusion 93 of the upper lateral panel 30 is disposed at or adjacent to the second end 116 of the second slot 94 of the lower body member 38. So configured, the first body edge portion 24 of the upper body member 16 is disposed at or adjacent to the second body edge portion 100 of the lower body member 38 and the second body edge portion 60 of the upper body member 16 is disposed at or adjacent to the first body edge portion 46 of the lower body member 38. In this second position (illustrated in Figure 8A, or when viewed along the upper body axis 18, the lower body axis 40, or parallel to the X-axis of the reference coordinate system), the cross-sectional shape of the upper body member 16 (i.e., the second cross-sectional assembly shape of the first cannula subassembly 12) and the cross-sectional shape of the lower body member 38 (i.e., the second cross-sectional assembly shape of the second cannula subassembly 14) cooperate to form a second cross-sectional assembly shape that is a circular cross-sectional shape. The first cannula subassembly 12 and the second cannula subassembly 14 may also have the shape of a circle when viewed along the upper body axis 18, the lower body axis 40, or parallel to the X-axis of the reference coordinate system (such as the view of Figures 8A).

In some embodiments, the first cannula subassembly 12 and the second cannula assembly 14 of the expandable cannula assembly 10 may be disposed in a third position (i.e., one or more intermediate positions, illustrated in Figure 8B) between the first position and the second position. In the third position(s), the first protrusion 124 of the lower lateral panel 50 is disposed between the first end 68 and the second end of the first slot 64 of the upper body member 16, and the second protrusion 126 of the lower lateral panel 50 is disposed between the first end 80 and the second end 82 of the second slot 76 of the upper body member 16. Also in the third position, the first protrusion 91 of the upper lateral panel 30 is disposed between the first end 108 and the second end 110 of the first slot 90 of the lower body member 38, and the second protrusion 93 of the upper lateral panel 30 is disposed between the first end 114 and the second end 116 of the second slot 94 of the lower body member 38.

In some embodiments, one or more resilient members (not shown) may be coupled to one or both of the first cannula subassembly 12 and the second cannula assembly 14 of the expandable cannula assembly 10, and the one or more resilient members may act on one or both of the first cannula subassembly 12 and the second cannula assembly 14 to bias the expandable cannula assembly 10 (*i.e.,* the first cannula subassembly 12 and the second cannula assembly 14) into the second position. In some embodiments, the one or more resilient members may be one or more elastic bands that surround the surface 28 of the upper body member 16 (see Fig. 8A) and the surface 51 of the lower body member 38 such that each of the one or more elastic bands surrounds the entire circumference of the upper body member 16 (see Fig. 8A) and the surface 51 of the lower body member 38 when the expandable cannula assembly 10 is in the second position. So positioned, the one or more elastic bands would apply a force to each of the upper body member 16 and the lower body member 38 that is substantially directed along or parallel to the Z-axis of the reference coordinate system, and this force would oppose the displacement of each of the upper body member 16 and the lower body member 38 from the second position into the first position.

In other embodiments, one or more springs or resilient members may be disposed between one or more portions of the upper body member 16 and the lower body member 38 to maintain or bias the upper body member 16 and the lower body member 38 (and the first cannula subassembly 12 and the second cannula assembly 14) into the second position.

In still other embodiments, all or a portion of the upper lateral panel 30 and the lower lateral panel 50 may be made from or comprise a resilient material that opposes the displacement of each of the upper body member 16 and the lower body member 38 from the second position into the first position. In such an embodiment, in the second position, the first protrusion 124 of the lower lateral panel 50 is disposed at or adjacent to the first end 68 of the first slot 64 of the upper body member 16, and the second protrusion 126 of the lower lateral panel 50 is disposed at or adjacent to the first end 80 of the second slot 76 of the upper body member 16. Also in the second position, the first protrusion 91 of the upper lateral panel 30 is disposed at or adjacent to the first end 108 of the first slot 90 of the lower body member 38, and the second protrusion 93 of the upper lateral panel 30 is disposed at or adjacent to the first end 114 of the second slot 94 of the lower body member 38. In such an embodiment, the position of the first and second protrusions 124, 126 of the lower lateral panel 50 and the first and protrusions 91, 93 of the upper lateral panel 30 do not change, but rather are retained in the first ends of the respective slots 64, 76, 90, 94 as the first cannula subassembly 12 and the second cannula assembly 14 are displaced from the second position into the first position. Thus, as the first cannula subassembly 12 and the second cannula assembly 14 are displaced from the second position into or towards the first (or third) position, the lower lateral panel 50 and the upper lateral panel 30 are stretched, and act to oppose the separating, or outward, displacement directed parallel to the Z-axis of the reference coordinate system. When the force causing the separating or outward displacement is removed, the lower lateral panel 50 and the upper lateral panel 30 act to return the first cannula subassembly 12 and the second cannula assembly 14 to or towards the second position.

When used during a procedure, the expandable cannula assembly 10 may be inserted into an incision of a patient in the second position to minimize trauma to the incision at the area of insertion. When a relatively large instrument 58 (illustrated in Fig. 11) is to be inserted through the expandable cannula assembly 10, a distal end of the instrument 58 is inserted into the proximal passage formed by the first end 20 of the upper body member 16 and the first end 42 of the lower body member 38. Although the first cannula subassembly 12 and the second cannula assembly 14 are illustrated in the first position prior to the insertion of the distal end of the instrument 58 in Fig. 11, contact between the distal end of the instrument 58 and the first end 20 of the upper body member 16 and the first end 42 of the lower body member 38 may cause the first cannula subassembly 12 and the second cannula assembly 14 to displace from the second position to the first position. While the expandable cannula assembly 10 is in the first position, the distal end of the instrument 58 may pass through the second end 22 of the upper body member 16 and the second end 44 of the lower body member 38 and be positioned at or adjacent to a desired treatment area.

When it is desired to remove the distal end of the instrument 58, the distal end of the instrument 58 may be displaced proximally until the distal end of the instrument 58 exits from the proximal passage formed by the first end 20 of the upper body member 16 and the first end 42 of the lower body member 38. In some embodiments (and particularly in embodiments with one or more resilient members acting one or both of the upper body member 16 and the lower body member 38), the lack of contact between the distal end of the instrument 58 and the first end 20 of the upper body member 16 and the first end 42 of the lower body member 38 may cause the first cannula subassembly 12 and the second cannula assembly 14 to displace from the first position to the second position. Accordingly, the time in which the expandable cannula assembly 10 is in the more traumatic first position is minimized, thereby correspondingly minimizing or avoiding trauma to the patient's incision.

In some embodiments, two or more expandable cannula assemblies 10 may be used and may be coupled such that the upper body axis 18a of the upper body member 16a of the first expandable cannula assembly 10a may be aligned with the upper body axis 18b of the upper body member 16b of the second expandable cannula assembly 10b, and so on. Accordingly, each segment of the overall assembly (*i.e.,* the first expandable cannula assembly 10a out of an assembly of a first, second, and third expandable cannula assembly 10a, 10b, 10c) may expand or deploy from the second position to the first position as the distal end of the instrument 58 passes through that particular segment.

Various advantages of an expandable cannula assembly have been discussed above. Embodiments discussed herein have been described by way of example in this specification. It will be apparent to those skilled in the art that the foregoing detailed disclosure is intended to be presented by way of example only, and is not limiting. Various alterations, improvements, and modifications will occur and are intended to those skilled in the art, though not expressly stated herein. These alterations, improvements, and modifications are intended to be suggested hereby, and are within the scope of the claimed invention as long as they fall within the scope of protection which is defined solely by the claims. The drawings included herein are not necessarily drawn to scale. Additionally, the recited order of processing elements or sequences, or the use of numbers, letters, or other designations therefore, is not intended to limit the claims to any order, except as may be specified in the claims. Accordingly, the invention is limited only by the following claims.

## Claims

1. An expandable cannula assembly (10) comprising:
a first cannula subassembly (12) comprising:
an upper body member (16, 16a, 16b) that extends along an upper body axis (18, 18a, 18b) from a first end (20) to a second end (22), the upper body member (16, 16a, 16b) having a first body edge portion (24) that extends parallel to and offset from the upper body axis (18, 18a, 18b), wherein a first upper body mating feature (26) is disposed along a first portion of a surface (28) of the upper body member (16, 16a, 16b);
an upper lateral panel (30) having a first panel edge (32) that is coupled to the upper body member (16, 16a, 16b) at or adjacent to the first body edge portion (24) of the upper body member (16, 16a, 16b), and wherein a first upper lateral panel mating feature (34) is disposed on a first portion of a surface (36) of the upper lateral panel (30); and
a second cannula subassembly (14) comprising:
a lower body member (38) that extends along a lower body axis (40) from a first end (42) to a second end (44), the lower body member (38) having a first body edge portion (46) that extends parallel to and offset from the lower body axis (40), wherein a first lower body mating feature (48) is disposed along a first portion of a surface (51) of the lower body member (38); and
a lower lateral panel (50) having a first panel edge (52) that is coupled to the lower body member (38) at or adjacent to the first body edge portion (46) of the lower body member (38), and wherein a first lower lateral panel mating feature (54) is disposed on a first portion of a surface (56) of the lower lateral panel (50);
wherein the first lower lateral panel mating feature (54) operatively engages the first upper body mating feature (26) such that the first lower lateral panel mating feature (54) is displaceable relative to the first upper body mating feature (26), and
the first upper lateral panel mating feature (34) operatively engages the first lower body mating feature (48) such that the first upper lateral panel mating feature (34) is displaceable relative to the first lower body mating feature (48); and
wherein the first cannula subassembly (12) and the second cannula assembly (14) are displaceable between a first position and a second position, wherein in the first position, the first cannula subassembly (12) and the second cannula assembly (14) have a first cross-sectional assembly shape and in the second position, the first cannula subassembly (12) and the second cannula assembly (14) have a second cross-sectional assembly shape that is different than the first cross-sectional shape;
wherein the first upper body mating feature (26) is a first slot (64) disposed along the first portion of the surface (28) of the upper body member (16, 16a, 16b), the first slot (64) of the upper body member (16, 16a, 16b) extending along a first slot axis (66) from a first end (68) to a second end (70),
wherein the first upper lateral panel mating feature (34) is a first protrusion (91) disposed on the first portion of the surface (36 ) of the upper lateral panel (30),
the first lower body mating feature (48) is a first slot (90) disposed along the first portion of the surface (50) of the lower body member (38), the first slot (90) of the lower body member (38) extending along a first slot axis (106) from a first end (108) to a second end (110), and
wherein the first lower lateral panel mating feature (54) is a first protrusion (124) disposed on the first portion of the surface (36) of the lower lateral panel (30), and
wherein the first protrusion (91) of the upper lateral panel (30) is slidably disposed within the first slot (90) of the lower body member (38), and wherein the first protrusion (124) of the lower lateral panel (50) is slidably disposed within the first slot (64) of the upper body member (16, 16a, 16b),
wherein the upper body member (16, 16a, 16b) includes a second body edge portion (60) that extends parallel to and offset from the upper body axis (18, 18a, 18b), and wherein the lower body member (38) includes a second body edge portion (100) that extends parallel to and offset from the lower body axis (40);
wherein in the second position, the first body edge portion (24) of the upper body member (16, 16a, 16b) is disposed at or adjacent to the second body edge portion (100) of the lower body member (38) and the second body edge portion (60) of the upper body member (16, 16a, 16b) is disposed at or adjacent to the first body edge portion (46) of the lower body member (38); and
wherein in the first position, the first body edge portion (24) of the upper body member (16, 16a, 16b) is disposed offset from the second body edge portion (100) of the lower body member (38) and the second body edge portion (60) of the upper body member (16, 16a, 16b) is disposed offset from the first body edge portion (46) of the lower body member (38).

2. The expandable cannula assembly (10) of claim 1, wherein:
(a) when the first cannula subassembly (12) and the second cannula assembly (14) are in the first position, the first protrusion (91) of the upper lateral panel (30) is disposed at or adjacent to the first end (108) of the first slot (90) of the lower body member (38) and the first protrusion (124) of the lower lateral panel (50) is disposed at or adjacent to the first end (68) of the first slot (64) of the upper body member (16, 16a, 16b), and
(b) when the first cannula subassembly (12) and the second cannula assembly (14) are in the second position, the first protrusion (91) of the upper lateral panel (30) is disposed at or adjacent to the second end (110) of the first slot (90) of the lower body member (38) and the first protrusion (124) of the lower lateral panel (50) is disposed at or adjacent to the second end (70) of the first slot (64) of the upper body member (16, 16a, 16b).

3. The expandable cannula assembly (10) of any one of claims 1 to 2, the upper lateral panel (30) further comprising a second panel edge, wherein each of the first panel edge (32) and the second panel edge of the upper lateral panel (30) extend parallel to and offset from the upper body axis (18, 18a, 18b), and
the lower lateral panel (50) further comprising a second panel edge, wherein each of the first panel edge (52) and the second panel edge of the lower lateral panel (50) extend parallel to and offset from the lower body axis (40).

4. The expandable cannula assembly (10) of claim 1, wherein the upper lateral panel (30) is made from a flexible material and/or wherein the lower lateral panel (50) is made from a flexible material.

5. The expandable cannula assembly (10) of claim 1, wherein the upper lateral panel (30) is configured to deform along an axis that is parallel to the upper body axis (18, 18a, 18b), and the lower lateral panel (50) is configured to deform along an axis that is parallel to the lower body axis (40).

6. The expandable cannula assembly (10) of claim 1, wherein when the first cannula subassembly (12) and the second cannula assembly (14) displace from the first position to the second position; and
wherein the upper body member (16, 16a, 16b) displaces in a direction normal to the upper body axis (18, 18a, 18b) or the lower body member (38) displaces in a direction normal to the lower body axis (40).

7. The expandable cannula assembly (10) of claim 1, wherein the upper body member (16, 16a, 16b) has a cross-sectional shape that is a segment of a first circle and the lower body member (38) has a cross-sectional shape that is a segment of a second circle, and in the second position, the second cross-sectional assembly shape is a circular cross-sectional shape.

8. The expandable cannula assembly (10) of claim 7, wherein in the first position, the cross-sectional shape of the upper body member (16, 16a, 16b) and the upper lateral panel (30) and the cross-sectional shape of the lower body member (38) and the lower lateral panel (50) cooperate to form a first cross-sectional assembly shape that is an ovular cross-sectional shape.

9. The expandable cannula assembly (10) of claim 1, wherein:
a second upper body mating feature (72) is disposed along a second portion of the surface (28) of the upper body member (16, 16a, 16b),
a second upper lateral panel mating feature (92) is disposed on a second portion of the surface (36) of the upper lateral panel (30),
a second lower body mating feature (96) is disposed along a second portion of the surface (51) of the lower body member (38),
a second lower lateral panel mating feature (74) is disposed on a second portion of the surface (56) of the lower lateral panel (50),
the second lower lateral panel mating feature (74) operatively engages the second upper body mating feature (72) such that the second lower lateral panel mating feature (74) is displaceable relative to the second upper body mating feature (72), and
the second upper lateral panel mating feature (92) operatively engages the second lower body mating feature (96) such that the second upper lateral panel mating feature (92) is displaceable relative to the second lower body mating feature (96).

10. The expandable cannula assembly (10) of claim 2, wherein:
a second upper body mating feature (72) is disposed along a second portion of the surface (28) of the upper body member (16, 16a, 16b),
a second upper lateral panel mating feature (92) is disposed on a second portion of the surface (36) of the upper lateral panel (30),
a second lower body mating feature (96) is disposed along a second portion of the surface (51) of the lower body member (38),
a second lower lateral panel mating feature (74) is disposed on a second portion of the surface (56) of the lower lateral panel (50),
the second lower lateral panel mating feature (74) operatively engages the second upper body mating feature (72) such that the second lower lateral panel mating feature (74) is displaceable relative to the second upper body mating feature (72), and
the second upper lateral panel mating feature (92) operatively engages the second lower body mating feature (96) such that the second upper lateral panel mating feature (92) is displaceable relative to the second lower body mating feature (96).

11. The expandable cannula assembly (10) of claim 10, wherein:
the second upper body mating feature (72) is a second slot (76) is disposed along the second portion of the surface (28) of the upper body member (16, 16a, 16b), the second slot (76) of the upper body member (16, 16a, 16b) extending along a second slot axis (78) from a first end (80) to a second end (82),
wherein the second upper lateral panel mating feature (92) is a second protrusion (93) disposed on the second portion of the surface (50) of the upper lateral panel (30),
the second lower body mating feature (96) is a second slot (94) is disposed along the second portion of the surface (50) of the lower body member (38), the second slot (94) of the lower body member (38) extending along a second slot axis (78, 112) from a first end (114) to a second end (116), and
wherein the second protrusion (93) of the upper lateral panel (30) is slidably disposed within the second slot (94) of the lower body member (38), and wherein the second protrusion (126) of the lower lateral panel (50) is slidably disposed within the second slot (76) of the upper body member (16, 16a, 16b), and
(a) when the first cannula subassembly (12) and the second cannula assembly (14) are in the second position, the second protrusion (93) of the upper lateral panel (30) is disposed at or adjacent to the second end (116) of the second slot (94) of the lower body member (38) and the second protrusion (93) of the lower lateral panel (50) is disposed at or adjacent to the second end (82) of the second slot (76) of the upper body member (16, 16a, 16b), and
(b) when the first cannula subassembly (12) and the second cannula assembly (14) are in the first position, the second protrusion (93) of the upper lateral panel (30) is disposed at or adjacent to the first end (114) of the second slot (94) of the lower body member (38) and the second protrusion (126) of the lower lateral panel (50) is disposed at or adjacent to the first end ( 80) of the second slot (76) of the upper body member (16, 16a, 16b).

## Patentansprüche

1. Expandierbare Kanülenanordnung (10), die Folgendes umfasst:
eine erste Kanülenunterbaugruppe (12), die Folgendes umfasst:
ein oberes Körperelement (16, 16a, 16b), das sich entlang einer oberen Körperachse (18, 18a, 18b) von einem ersten Ende (20) zu einem zweiten Ende (22) erstreckt, wobei das obere Körperelement (16, 16a, 16b) einen ersten Körperrandabschnitt (24) aufweist, der sich parallel zu und versetzt von der oberen Körperachse (18, 18a, 18b) erstreckt, wobei ein erstes oberer Körper-Passungsmerkmal (26) entlang eines ersten Abschnitts einer Oberfläche (28) des oberen Körperelements (16, 16a, 16b) angeordnet ist;
ein oberes seitliches Paneel (30) mit einer ersten Paneelkante (32), die mit dem oberen Körperelement (16, 16a, 16b) an oder angrenzend an den ersten Körperrandabschnitt (24) des oberen Körperelements (16, 16a, 16b) gekoppelt ist, und, wobei ein erstes oberes seitliches Paneel-Passungsmerkmal (34) an einem ersten Abschnitt einer Oberfläche (36) des oberen seitlichen Paneels (30) angeordnet ist; und eine zweite Kanülenunterbaugruppe (14), die Folgendes umfasst:
ein unteres Körperelement (38), das sich entlang einer unteren Körperachse (40) von einem ersten Ende (42) zu einem zweiten Ende (44) erstreckt, wobei das untere Körperelement (38) einen ersten Körperrandabschnitt (46) aufweist, der sich parallel zu und versetzt von der unteren Körperachse (40) erstreckt, wobei ein erstes unteres Körper-Passungsmerkmal (48) entlang eines ersten Abschnitts einer Oberfläche (51) des unteren Körperelements (38) angeordnet ist; und
ein unteres seitliches Paneel (50) mit einer ersten Paneelkante (52), die mit dem unteren Körperelement (38) an oder angrenzend an den ersten Körperrandabschnitt (46) des unteren Körperelements (38) gekoppelt ist, und wobei ein erstes unteres seitliches Paneel-Passungsmerkmal (54) auf einem ersten Abschnitt einer Oberfläche (56) des unteren seitlichen Paneels (50) angeordnet ist;
wobei das erste untere seitliche Paneel-Passungsmerkmal (54) funktionsmäßig mit dem ersten oberen Körper-Passungsmerkmal (26) in Eingriff steht, so dass das erste untere seitliche Paneel-Passungsmerkmal (54) relativ zu dem ersten oberen Körper-Passungsmerkmal (26) verschiebbar ist, und
das erste obere seitliche Paneel-Passungsmerkmal (34) funktionsmäßig mit dem ersten unteren Körper-Passungsmerkmal (48) in Eingriff steht, so dass das erste obere seitliche Paneel-Passungsmerkmal (34) relativ zu dem ersten unteren Körper-Passungsmerkmal (48) verschiebbar ist; und
wobei die erste Kanülenunterbaugruppe (12) und die zweite Kanülenbaugruppe (14) zwischen einer ersten Position und einer zweiten Position verschiebbar sind, wobei in der ersten Position die erste Kanülenunterbaugruppe (12) und die zweite Kanülenbaugruppe (14) eine erste Querschnittsbaugruppenform aufweisen und in der zweiten Position die erste Kanülenunterbaugruppe (12) und die zweite Kanülenbaugruppe (14) eine zweite Querschnittsbaugruppenform aufweisen, die sich von der ersten Querschnittsform unterscheidet;
wobei das erste obere Körper-Passungsmerkmal (26) ein erster Schlitz (64) ist, der entlang des ersten Abschnitts der Oberfläche (28) des oberen Körperelements (16, 16a, 16b) angeordnet ist, wobei sich der erste Schlitz (64) des oberen Körperelements (16, 16a, 16b) entlang einer ersten Schlitzachse (66) von einem ersten Ende (68) zu einem zweiten Ende (70) erstreckt,
wobei das erste obere seitliche Paneel-Passungsmerkmal (34) ein erster Vorsprung (91) ist, der auf dem ersten Abschnitt der Oberfläche (36) des oberen seitlichen Paneels (30) angeordnet ist,
wobei das erste untere Körper-Passungsmerkmal (48) ein erster Schlitz (90) ist, der entlang des ersten Abschnitts der Oberfläche (50) des unteren Körperelements (38) angeordnet ist, wobei sich der erste Schlitz (90) des unteren Körperelements (38) entlang einer ersten Schlitzachse (106) von einem ersten Ende (108) zu einem zweiten Ende (110) erstreckt, und
wobei das erste untere seitliche Paneel-Passungsmerkmal (54) ein erster Vorsprung (124) ist, der auf dem ersten Abschnitt der Oberfläche (36) des unteren seitlichen Paneels (30) angeordnet ist, und
wobei der erste Vorsprung (91) des oberen seitlichen Paneels (30) verschiebbar innerhalb des ersten Schlitzes (90) des unteren Körperelements (38) angeordnet ist, und wobei der erste Vorsprung (124) des unteren seitlichen Paneels (50) verschiebbar innerhalb des ersten Schlitzes (64) des oberen Körperelements (16, 16a, 16b) angeordnet ist,
wobei das obere Körperelement (16, 16a, 16b) einen zweiten Körperrandabschnitt (60) aufweist, der sich parallel zu und versetzt von der oberen Körperachse (18, 18a, 18b) erstreckt, und wobei das untere Körperelement (38) einen zweiten Körperrandabschnitt (100) aufweist, der sich parallel zu und versetzt von der unteren Körperachse (40) erstreckt;
wobei in der zweiten Position der erste Körperrandabschnitt (24) des oberen Körperelements (16, 16a, 16b) an oder benachbart zu dem zweiten Körperrandabschnitt (100) des unteren Körperelements (38) angeordnet ist und der zweite Körperrandabschnitt (60) des oberen Körperelements (16, 16a, 16b) an oder benachbart zu dem ersten Körperrandabschnitt (46) des unteren Körperelements (38) angeordnet ist; und
wobei in der ersten Position der erste Körperrandabschnitt (24) des oberen Körperelements (16, 16a, 16b) ) gegenüber dem zweiten Körperrandabschnitt (100) des unteren Körperelements (38) versetzt angeordnet ist und der zweite Körperrandabschnitt (60) des oberen Körperelements (16, 16a, 16b) gegenüber dem ersten Körperrandabschnitt (46) des unteren Körperelements (38) versetzt angeordnet ist.

2. Expandierbare Kanülenanordnung (10) nach Anspruch 1, wobei:
(a) wenn sich die erste Kanülenunterbaugruppe (12) und die zweite Kanülenbaugruppe (14) in der ersten Position befinden, der erste Vorsprung (91) des oberen seitlichen Paneels (30) am oder neben dem ersten Ende (108) des ersten Schlitzes (90) des unteren Körperelements (38) angeordnet ist und der erste Vorsprung (124) des unteren seitlichen Paneels (50) am oder neben dem ersten Ende (68) des ersten Schlitzes (64) des oberen Körperelements (16, 16a, 16b) angeordnet ist, und
(b) wenn sich die erste Kanülenunterbaugruppe (12) und die zweite Kanülenbaugruppe (14) in der zweiten Position befinden, der erste Vorsprung (91) des oberen seitlichen Paneels (30) am oder neben dem zweiten Ende (110) des ersten Schlitzes (90) des unteren Körperelements (38) angeordnet ist und der erste Vorsprung (124) des unteren seitlichen Paneels (50) am oder neben dem zweiten Ende (70) des ersten Schlitzes (64) des oberen Körperelements (16, 16a, 16b) angeordnet ist.

3. Expandierbare Kanülenanordnung (10) nach einem der Ansprüche 1 bis 2, wobei das obere seitliche Paneel (30) ferner eine zweite Paneelkante aufweist, wobei sich sowohl die erste Paneelkante (32) als auch die zweite Paneelkante des oberen seitlichen Paneels (30) parallel zu und versetzt von der oberen Körperachse (18, 18a, 18b) erstrecken, und
das untere seitliche Paneel (50) ferner eine zweite Paneelkante umfasst, wobei sich sowohl die erste Paneelkante (52) als auch die zweite Paneelkante des unteren seitlichen Paneels (50) parallel zu und versetzt von der unteren Körperachse (40) erstrecken.

4. Expandierbare Kanülenanordnung (10) nach Anspruch 1, wobei das obere seitliche Paneel (30) aus einem flexiblen Material hergestellt ist und/oder wobei das untere seitliche Paneel (50) aus einem flexiblen Material hergestellt ist.

5. Expandierbare Kanülenanordnung (10) nach Anspruch 1, wobei das obere seitliche Paneel (30) so ausgestaltet ist, dass es sich entlang einer Achse verformt, die parallel zur oberen Körperachse (18, 18a, 18b) ist, und das untere seitliche Paneel (50) so ausgestaltet ist, dass es sich entlang einer Achse verformt, die parallel zur unteren Körperachse (40) ist.

6. Expandierbare Kanülenbaugruppe (10) nach Anspruch 1, wobei, wenn sich die erste Kanülenunterbaugruppe (12) und die zweite Kanülenbaugruppe (14) von der ersten Position in die zweite Position verschieben; und
wobei das obere Körperelement (16, 16a, 16b) sich in einer Richtung senkrecht zur oberen Körperachse (18, 18a, 18b) oder das untere Körperelement (38) sich in einer Richtung senkrecht zur unteren Körperachse (40) verschiebt.

7. Expandierbare Kanülenanordnung (10) nach Anspruch 1, wobei das obere Körperelement (16, 16a, 16b) eine Querschnittsform aufweist, die ein Segment eines ersten Kreises ist, und das untere Körperelement (38) eine Querschnittsform aufweist, die ein Segment eines zweiten Kreises ist, und in der zweiten Position die zweite Querschnittsbaugruppenform eine kreisförmige Querschnittsform ist.

8. Expandierbare Kanülenanordnung (10) nach Anspruch 7, wobei in der ersten Position die Querschnittsform des oberen Körperelements (16, 16a, 16b) und des oberen seitliche Paneels (30) und die Querschnittsform des unteren Körperelements (38) und des unteren seitlichen Paneels (50) zusammenwirken, um eine erste Querschnittsbaugruppenform zu bilden, die eine ovale Querschnittsform ist.

9. Expandierbare Kanülenanordnung (10) nach Anspruch 1, wobei:
ein zweites oberer Körper-Passungsmerkmal (72) entlang eines zweiten Abschnitts der Oberfläche (28) des oberen Körperelements (16, 16a, 16b) angeordnet ist,
ein zweites oberes seitliches Paneel-Passungsmerkmal (92) auf einem zweiten Abschnitt der Oberfläche (36) des oberen seitlichen Paneels (30) angeordnet ist,
ein zweites unteres Körper-Passungsmerkmal (96) entlang eines zweiten Abschnitts der Oberfläche (51) des unteren Körperteils (38) angeordnet ist,
ein zweites unteres seitliches Paneel-Passungsmerkmal (74) auf einem zweiten Abschnitt der Oberfläche (56) des unteren seitlichen Paneels (50) angeordnet ist,
das zweite untere seitliche Paneel-Passungsmerkmal (74) funktionsmäßig mit dem zweiten oberen Körper-Passungsmerkmal (72) in Eingriff steht, so dass das zweite untere seitliche Paneel-Passungsmerkmal (74) relativ zu dem zweiten oberen Körper-Passungsmerkmal (72) verschiebbar ist, und
das zweite obere seitliche Paneel-Passungsmerkmal (92) funktionsmäßig mit dem zweiten unteren Körper-Passungsmerkmal (96) in Eingriff steht, so dass das zweite obere seitliche Paneel-Passungsmerkmal (92) relativ zu dem zweiten unteren Körper-Passungsmerkmal (96) verschiebbar ist.

10. Expandierbare Kanülenanordnung (10) nach Anspruch 2, wobei:
ein zweites oberes Körper-Passungsmerkmal (72) entlang eines zweiten Abschnitts der Oberfläche (28) von des oberen Körperelements (16, 16a, 16b) angeordnet ist,
ein zweites oberes seitliches Paneel-Passungsmerkmal (92) auf einem zweiten Abschnitt der Oberfläche (36) der oberen Seitenwand (30) angeordnet ist,
ein zweites unteres Körper-Passungsmerkmal (96) entlang eines zweiten Abschnitts der Oberfläche (51) des unteren Körperteils (38) angeordnet ist,
ein zweites unteres seitliches Paneel-Passungsmerkmal (74) auf einem zweiten Abschnitt der Oberfläche (56) der unteren Seitenwand (50) angeordnet ist,
das zweite untere seitliche seitliches Paneel-Passungsmerkmal (74) funktionsmäßig mit dem zweiten oberen Körper-Passungsmerkmal (72) in Eingriff steht, so dass das zweite untere seitliche Paneel-Passungsmerkmal (74) relativ zu dem zweiten oberen Körper-Passungsmerkmal (72) verschiebbar ist, und
das zweite obere seitliche Paneel-Passungsmerkmal (92) funktionsmäßig in das zweite untere Körper-Passungsmerkmal (96) eingreift, so dass das zweite obere seitliche Paneel-Passungsmerkmal (92) relativ zu dem zweiten unteren Körper-Passungsmerkmal (96) verschiebbar ist.

11. Expandierbare Kanülenanordnung (10) nach Anspruch 10, wobei:
das zweite obere Körper-Passungsmerkmal (72) ein zweiter Schlitz (76) ist, der entlang des zweiten Abschnitts der Oberfläche (28) des oberen Körperelements (16, 16a, 16b) angeordnet ist, wobei sich der zweite Schlitz (76) des oberen Körperelements (16, 16a, 16b) entlang einer zweiten Schlitzachse (78) von einem ersten Ende (80) zu einem zweiten Ende (82) erstreckt,
das zweite obere seitliche Paneel-Passungsmerkmal (92) ein zweiter Vorsprung (93) ist, der auf dem zweiten Abschnitt der Oberfläche (50) des oberen seitlichen Paneels (30) angeordnet ist,
das zweite untere Körper-Passungsmerkmal (96) ein zweiter Schlitz (94) ist, der entlang des zweiten Abschnitts der Oberfläche (50) des unteren Körperelements (38) angeordnet ist, wobei sich der zweite Schlitz (94) des unteren Körperelements (38) entlang einer zweiten Schlitzachse (78, 112) von einem ersten Ende (114) zu einem zweiten Ende (116) erstreckt, und
der zweite Vorsprung (93) des oberen seitlichen Paneels (30) gleitend in dem zweiten Schlitz (94) des unteren Körperelements (38) angeordnet ist, und wobei der zweite Vorsprung (126) des unteren seitlichen Paneels (50) gleitend in dem zweiten Schlitz (76) des oberen Körperelements (16, 16a, 16b) angeordnet ist, und
(a) wenn sich die erste Kanülenunterbaugruppe (12) und die zweite Kanülenbaugruppe (14) in der zweiten Position befinden, der zweite Vorsprung (93) des oberen seitlichen Paneels (30) an oder neben dem zweiten Ende (116) des zweiten Schlitzes (94) des unteren Körperelements (38) angeordnet ist und der zweite Vorsprung (93) des unteren seitlichen Paneels (50) an oder neben dem zweiten Ende (82) des zweiten Schlitzes (76) des oberen Körperelements (16, 16a, 16b) angeordnet ist, und
(b) wenn sich die erste Kanülenunterbaugruppe (12) und die zweite Kanülenbaugruppe (14) in der ersten Position befinden, der zweite Vorsprung (93) des oberen seitlichen Paneels (30) am oder neben dem ersten Ende (114) des zweiten Schlitzes (94) des unteren Körperelements (38) angeordnet ist und der zweite Vorsprung (126) des unteren seitlichen Paneels (50) am oder neben dem ersten Ende (80) des zweiten Schlitzes (76) des oberen Körperelements (16, 16a, 16b) angeordnet ist.

## Revendications

1. Un ensemble de canules extensibles (10) comprenant :
un premier sous-ensemble de canule (12) comprenant :
un élément de corps supérieur (16, 16a, 16b) qui s'étend le long d'un axe de corps supérieur (18, 18a, 18b) d'une première extrémité (20) à une deuxième extrémité (22), l'élément de corps supérieur (16, 16a, 16b) ayant une première partie de bord de corps (24) qui s'étend parallèlement à l'axe du corps supérieur (18, 18a, 18b) et qui est décalée par rapport à celui-ci, dans lequel une première caractéristique d'accouplement du corps supérieur (26) est disposée le long d'une première partie d'une surface (28) de l'élément du corps supérieur (16, 16a, 16b) ;
un panneau latéral supérieur (30) ayant un premier bord de panneau (32) qui est couplé à l'élément de corps supérieur (16, 16a, 16b) au niveau ou à côté de la première partie de bord de corps (24) de l'élément de corps supérieur (16, 16a, 16b), et dans lequel une première caractéristique d'accouplement de panneau latéral supérieur (34) est disposée sur une première partie d'une surface (36) du panneau latéral supérieur (30) ; et
un deuxième sous-ensemble de canule (14) comprenant :
un élément inférieur du corps (38) qui s'étend le long d'un axe inférieur du corps (40) d'une première extrémité (42) à une deuxième extrémité (44), l'élément inférieur du corps (38) ayant une première partie de bord du corps (46) qui s'étend parallèlement à l'axe inférieur du corps (40) et en décalage par rapport à celui-ci, dans lequel un première élément d'adaptation du corps inférieur (48) est disposée le long d'une première partie d'une surface (51) de l'élément inférieur du corps (38) ; et
un panneau latéral inférieur (50) ayant un premier bord de panneau (52) qui est couplé à l'élément inférieur du corps (38) au niveau ou à côté de la première partie du bord du corps (46) de l'élément inférieur du corps (38), et dans lequel une première caractéristique d'accouplement du panneau latéral inférieur (54) est disposée sur une première partie d'une surface (56) du panneau latéral inférieur (50) ;
dans lequel le premier élément de fixation du panneau latéral inférieur (54) s'engage de manière opérationnelle dans le premier élément de fixation du corps supérieur (26) de sorte que le premier élément de fixation du panneau latéral inférieur (54) est déplaçable par rapport au premier élément de fixation du corps supérieur (26), et
le premier élément de fixation du panneau latéral supérieur (34) est en prise avec le premier élément d'adaptation du corps inférieur (48) de sorte que le premier élément de fixation du panneau latéral supérieur (34) peut être déplacé par rapport au premier élément d'adaptation du corps inférieur (48) ; et
dans lequel le premier sous-ensemble de canule (12) et le deuxième ensemble de canule (14) peuvent être déplacés entre une première position et une deuxième position, dans laquelle, dans la première position, le premier sous-ensemble de canule (12) et le deuxième ensemble de canule (14) ont une première forme de section transversale et dans la deuxième position, le premier sous-ensemble de canule (12) et le deuxième ensemble de canule (14) ont une deuxième forme de section transversale qui est différente de la première forme de section transversale
dans lequel la première caractéristique d'accouplement du corps supérieur (26) est une première fente (64) disposée le long de la première partie de la surface (28) de l'élément du corps supérieur (16, 16a, 16b), la première fente (64) de l'élément du corps supérieur (16, 16a, 16b) s'étendant le long d'un premier axe de fente (66) d'une première extrémité (68) à une seconde extrémité (70),
dans lequel la première caractéristique d'accouplement du panneau latéral supérieur (34) est une première protubérance (91) disposée sur la première partie de la surface (36 ) du panneau latéral supérieur (30),
le premier élément d'adaptation du corps inférieur (48) est une première fente (90) disposée le long de la première partie de la surface (50) de l'élément inférieur (38), la première fente (90) de l'élément inférieur (38) s'étendant le long d'un premier axe de fente (106) d'une première extrémité (108) à une seconde extrémité (110), et
dans lequel la première caractéristique d'accouplement du panneau latéral inférieur (54) est une première protubérance (124) disposée sur la première partie de la surface (36) du panneau latéral inférieur (30), et
dans lequel la première protubérance (91) du panneau latéral supérieur (30) est disposée de manière coulissante dans la première fente (90) de l'élément inférieur du corps (38), et dans lequel la première protubérance (124) du panneau latéral inférieur (50) est disposée de manière coulissante dans la première fente (64) de l'élément supérieur du corps (16, 16a, 16b),
dans lequel l'élément supérieur du corps (16, 16a, 16b) comprend une deuxième partie du bord du corps (60) qui s'étend parallèlement à l'axe du corps supérieur (18, 18a, 18b) et qui en est décalée, et dans lequel l'élément inférieur du corps (38) comprend une deuxième partie du bord du corps (100) qui s'étend parallèlement à l'axe du corps inférieur (40) et qui en est décalée ;
dans laquelle, dans la deuxième position, la première partie du bord du corps (24) de l'élément supérieur du corps (16, 16a, 16b) est disposée au niveau de la deuxième partie du bord du corps (100) de l'élément inférieur du corps (38) ou à proximité de celle-ci, et la deuxième partie du bord du corps (60) de l'élément supérieur du corps (16, 16a, 16b) est disposée au niveau de la première partie du bord du corps (46) de l'élément inférieur du corps (38) ou à proximité de celle-ci ; et
dans laquelle, dans la première position, la première partie du bord du corps (24) de l'élément supérieur du corps (16, 16a, 16b) est décalée par rapport à la deuxième partie du bord du corps (100) de l'élément inférieur du corps (38) et la deuxième partie du bord du corps (60) de l'élément supérieur du corps (16, 16a, 16b) est décalée par rapport à la première partie du bord du corps (46) de l'élément inférieur du corps (38).

2. L'ensemble canule extensible (10) de la revendication 1, dans laquelle :
(a) lorsque le premier sous-ensemble de canule (12) et le deuxième ensemble de canule (14) sont dans la première position, la première protubérance (91) du panneau latéral supérieur (30) est disposée à la première extrémité (108) de la première fente (90) de l'élément inférieur du corps (38) ou à proximité de celle-ci, et la première protubérance (124) du panneau latéral inférieur (50) est disposée à la première extrémité (68) de la première fente (64) de l'élément supérieur du corps (16, 16a, 16b) ou à proximité de celle-ci, et
(b) lorsque le premier sous-ensemble de canule (12) et le deuxième ensemble de canule (14) sont dans la deuxième position, la première protubérance (91) du panneau latéral supérieur (30) est disposée à la deuxième extrémité (110) de la première fente (90) de l'élément inférieur du corps (38) ou à proximité de celle-ci, et la première protubérance (124) du panneau latéral inférieur (50) est disposée à la deuxième extrémité (70) de la première fente (64) de l'élément supérieur du corps (16, 16a, 16b) ou à proximité de celle-ci.

3. L'ensemble de canule extensible (10) de l'une des revendications 1 à 2, le panneau latéral supérieur (30) comprenant en outre un deuxième bord de panneau, dans lequel le premier bord de panneau (32) et le deuxième bord de panneau du panneau latéral supérieur (30) s'étendent parallèlement à l'axe du corps supérieur (18, 18a, 18b) et sont décalés par rapport à cet axe, et
le panneau latéral inférieur (50) comprend en outre un deuxième bord de panneau, dans lequel le premier bord de panneau (52) et le deuxième bord de panneau du panneau latéral inférieur (50) s'étendent parallèlement à l'axe de la partie inférieure du corps (40) et sont décalés par rapport à celui-ci.

4. L'ensemble canule extensible de la revendication 1, dans laquelle le panneau latéral supérieur (30) est fait d'un matériau flexible et/ou dans laquelle le panneau latéral inférieur (50) est fait d'un matériau flexible.

5. L'ensemble de canule extensible (10) de la revendication 1, dans lequel le panneau latéral supérieur (30) est configuré pour se déformer le long d'un axe parallèle à l'axe du corps supérieur (18, 18a, 18b), et le panneau latéral inférieur (50) est configuré pour se déformer le long d'un axe parallèle à l'axe du corps inférieur (40).

6. L'ensemble canule extensible (10) de la revendication 1, dans lequel lorsque le premier sous-ensemble canule (12) et le deuxième ensemble canule (14) se déplacent de la première position à la deuxième position ; et
dans lequel l'élément supérieur du corps (16, 16a, 16b) se déplace dans une direction normale à l'axe supérieur du corps (18, 18a, 18b) ou l'élément inférieur du corps (38) se déplace dans une direction normale à l'axe inférieur du corps (40).

7. L'ensemble canule extensible (10) de la revendication 1, dans laquelle l'élément supérieur (16, 16a, 16b) a une forme de section transversale qui est un segment d'un premier cercle et l'élément inférieur (38) a une forme de section transversale qui est un segment d'un second cercle, et dans la seconde position, la seconde forme de section transversale de l'assemblage est une forme de section transversale circulaire.

8. L'ensemble canule extensible (10) de la revendication 7, dans laquelle, dans la première position, la forme de la section transversale de l'élément supérieur (16, 16a, 16b) et du panneau latéral supérieur (30) et la forme de la section transversale de l'élément inférieur (38) et du panneau latéral inférieur (50) coopèrent pour former une première forme de section transversale de l'assemblage qui est une forme de section transversale ovoïde.

9. L'ensemble canule extensible (10) de la revendication 1, dans laquelle :
un deuxième élément d'accouplement du corps supérieur (72) est disposé le long d'une deuxième partie de la surface (28) de l'élément du corps supérieur (16, 16a, 16b),
un deuxième élément d'accouplement du panneau latéral supérieur (92) est disposé sur une deuxième partie de la surface (36) du panneau latéral supérieur (30),
un deuxième élément d'accouplement (96) du corps inférieur est disposé le long d'une deuxième partie de la surface (51) de l'élément inférieur (38),
un deuxième élément d'accouplement du panneau latéral inférieur (74) est disposé sur une deuxième partie de la surface (56) du panneau latéral inférieur (50),
le deuxième élément de fixation (74) du panneau latéral inférieur est en prise avec le deuxième élément de fixation (72) du corps supérieur de sorte que le deuxième élément de fixation (74) du panneau latéral inférieur peut être déplacé par rapport au deuxième élément de fixation (72) du corps supérieur, et
le deuxième élément de fixation (92) du panneau latéral supérieur est en prise avec le deuxième élément de fixation (96) du corps inférieur, de sorte que le deuxième élément de fixation (92) du panneau latéral supérieur peut être déplacé par rapport au deuxième élément de fixation (96) du corps inférieur.

10. L'ensemble canule extensible de la revendication 2, dans laquelle :
une deuxième caractéristique d'accouplement du corps supérieur (72) est disposée le long d'une deuxième partie de la surface (28) de l'élément du corps supérieur (16, 16a, 16b),
un deuxième élément d'accouplement du panneau latéral supérieur (92) est disposé sur une deuxième partie de la surface (36) du panneau latéral supérieur (30),
un deuxième élément d'accouplement (96) du corps inférieur est disposé le long d'une deuxième partie de la surface (51) de l'élément inférieur (38),
un deuxième élément d'accouplement du panneau latéral inférieur (74) est disposé sur une deuxième partie de la surface (56) du panneau latéral inférieur (50),
le deuxième élément de fixation (74) du panneau latéral inférieur est en prise avec le deuxième élément de fixation (72) du corps supérieur de sorte que le deuxième élément de fixation (74) du panneau latéral inférieur peut être déplacé par rapport au deuxième élément de fixation (72) du corps supérieur, et
le deuxième élément de fixation (92) du panneau latéral supérieur est en prise avec le deuxième élément de fixation (96) du corps inférieur, de sorte que le deuxième élément de fixation (92) du panneau latéral supérieur peut être déplacé par rapport au deuxième élément de fixation (96) du corps inférieur.

11. L'ensemble canule extensible de la revendication 10, dans laquelle :
le deuxième élément d'adaptation du corps supérieur (72) est une deuxième fente (76) disposée le long de la deuxième partie de la surface (28) de l'élément du corps supérieur (16, 16a, 16b), la deuxième fente (76) de l'élément du corps supérieur (16, 16a, 16b) s'étendant le long d'un deuxième axe de fente (78) depuis une première extrémité (80) jusqu'à une deuxième extrémité (82),
dans lequel la deuxième caractéristique d'accouplement du panneau latéral supérieur (92) est une deuxième protubérance (93) disposée sur la deuxième partie de la surface (50) du panneau latéral supérieur (30),
le deuxième élément d'accouplement (96) du corps inférieur est une deuxième fente (94) disposée le long de la deuxième partie de la surface (50) de l'élément inférieur (38), la deuxième fente (94) de l'élément inférieur (38) s'étendant le long d'un deuxième axe de fente (78, 112) d'une première extrémité (114) à une deuxième extrémité (116), et
dans lequel la deuxième protubérance (93) du panneau latéral supérieur (30) est disposée de manière coulissante dans la deuxième fente (94) de l'élément inférieur du corps (38), et dans lequel la deuxième protubérance (126) du panneau latéral inférieur (50) est disposée de manière coulissante dans la deuxième fente (76) de l'élément supérieur du corps (16, 16a, 16b), et
(a) lorsque le premier sous-ensemble de canule (12) et le deuxième ensemble de canule (14) sont dans la deuxième position, la deuxième protubérance (93) du panneau latéral supérieur (30) est disposée à la deuxième extrémité (116) de la deuxième fente (94) de l'élément inférieur du corps (38) ou à proximité de celle-ci et la deuxième protubérance (93) du panneau latéral inférieur (50) est disposée à la deuxième extrémité (82) de la deuxième fente (76) de l'élément supérieur du corps (16, 16a, 16b) ou à proximité de celle-ci, et
(b) lorsque le premier sous-ensemble de canule (12) et le deuxième ensemble de canule (14) sont dans la première position, la deuxième protubérance (93) du panneau latéral supérieur (30) est disposée à la première extrémité (114) de la deuxième fente (94) de l'élément inférieur du corps (38) ou à proximité de celle-ci, et la deuxième protubérance (126) du panneau latéral inférieur (50) est disposée à la première extrémité ( 80) de la deuxième fente (76) de l'élément supérieur du corps (16, 16a, 16b) ou à proximité de celle-ci.
